# EUROPEAN PATENT APPLICATION

(11) **EP 4 846 983 A2**
(43) Date of publication of application: **02.12.2026**
(21) Application number: 26211926.6
(22) Date of filing: 01.08.2024
(51) Int. Cl.: A61F 2/66

(54) **AN INTELLIGENT ADAPTIVE CONTROL SYSTEM FOR PROSTHETIC, ORTHOSIS OR ARTIFICIAL LIMBS**

(30) Priority: 03.08.2023 NL 2035543
(62) Document number(s) of the earlier application(s) in accordance with Art. 76 EPC: 24752026.5 / 4 753 627
(71) Applicant: Axiles Bionics BV, 1130 Brussel (BE)
(72) Inventor: CHERELLE, Pierre Philippe, 1130 Brussel (BE); GOMEZ, Felipe M., 1130 Brussel (BE); ENGELMAN, David, 1130 Brussel (BE)
(74) Representative: Arnold & Siedsma

(57) **Abstract**

A method for development of a favored control agent for a prosthesis, orthosis, or artificial limb, comprising the steps of:
- obtaining a motion model of the prosthesis, orthosis, or artificial limb;
- obtaining a plurality of training response models of the prosthesis, orthosis, or artificial limb;
- obtaining an initial plurality of control agents, each being associated to a different training response model of the plurality of training response models;
- selecting a control agent among the initial plurality of control agents, and defining the selected control agent as the favored control agent;
and, repeatedly:
- selecting a challenger control agent among the plurality of control agents;
- obtaining a user input indicative of a user preference among the challenger control agent and the favored control agent;
- based on the user input, selecting a new favored control agent between the previous favored control agent and the challenger control agent.

## Description

### FIELD OF INVENTION

The field of the invention relates generally to systems and methods for prostheses, orthoses, or artificial limbs enabling control thereof using specifically developed models based on machine learning and reinforcement learning. Particular embodiments relate to matters pertaining to prostheses or orthoses for functionally assisting, enhancing, and/or replacing a limb of a human or animal subject or for augmenting a body or a part of a body of a human or animal subject and to matters pertaining to artificial limbs for functionally acting as a limb of a humanoid or animal-inspired robot.

### BACKGROUND

In existing prostheses, orthoses, or artificial limbs, collecting data using sensors related to the operation of the prosthesis, orthosis, or artificial limb is known to be able to obtain feedback during use. The collected data can be taken into account in order to be able to more accurately control the operation of the prosthesis, orthosis, or artificial limb. This is especially the case with prostheses, orthoses, or artificial limbs including actuators such as motors, or dampers. Body motion (human or animal) involves the coordination of various interrelated and complex translations and rotations of different body parts. For a user of a prosthesis or orthosis to have a more comfortable experience during use, or for an artificial limb to recreate organic body motion, closer to a natural body motion, monitoring and collecting data is necessary. After collecting data, processing of said data to actively or passively control the different elements of the prosthesis, orthosis, or artificial limb allows to emulate motions of the corresponding body part. However, the active or passive control of prostheses, orthoses, or artificial limbs still depends on a predetermined set of configuration and/or behavioral data associated to the elements of the prosthesis, orthosis, or artificial limb, and has limited adaptive capabilities relative to environmental changes or behavior changes which would occur in reality.

In prior art solutions, to address the above-mentioned problems, various predetermined sets of configuration and/or behavioral data associated with a fixed number of environmental settings and/or behaviors are used. However, such ways of controlling the prosthesis, orthosis, or artificial limb are limited in terms of adaptability and, especially when transitioning between different environments or behaviors, precise control of the prosthesis, orthosis, or artificial limb to approximate natural body motion is still lacking.

### SUMMARY

The object of embodiments of the invention is to provide, improve, and/or develop control agents, preferably based on machine learning, *e.g.* using deep learning, probabilistic models, gaussian processes, and reinforcement learning, to be used in controlling a prosthesis, orthosis, or artificial limb which can more easily and accurately adapt to environmental disturbances such as speed transients and terrain variation, as well as to activity (or behavior) changes.

According to a first aspect of the invention, there is provided a system comprising a prosthesis, orthosis, or artificial limb. The system comprises at least one sensing means, and a state determining means. The at least one sensing means is configured to sense data related to a motion performed by a user involving the prosthesis, orthosis, or artificial limb worn or equipped by said user. The state determining means is configured to repeatedly perform the following steps: determine a state associated to the motion based on the sensed data, said sensed data being obtained from the at least one sensing means; and obtain an uncertainty value representative for a level of uncertainty that the user is in the determined state.

By determining the state associated with the motion as well as the uncertainty value, a control agent used in controlling the prosthesis, orthosis, or artificial limb can base its output on the state and the uncertainty value to more accurately allow for the prosthesis, orthosis, or artificial limb to make motions mimicking an equivalent natural body motion. Instead of being based on a fixed number of sets of predetermined configuration and/or behavioral data, and switch from one to the other depending on whether the motion indicates that the user wearing the prosthesis, orthosis or equipped with the artificial limb has switched from one activity to the next, for example, the control of the prosthesis, orthosis, or artificial limb can be adapted continuously by the repeated determination of the state, thereby providing a smoother adaptation to the switch in activity or transition from one activity to the next. Additionally, the uncertainty value which is obtained can be used as a safety mechanism to avoid incorrect control of the prosthesis, orthosis, or artificial limb due to incorrect (or false) transitioning triggers.

In the context of the invention the term orthosis is used to refer both to a device used to assist a person with a limb pathology, as well as to a device that augments the performance of an able-bodied wearer. The latter is also called an exoskeleton. Thus, when referring to an orthosis in the context of this application, this could be an orthosis used to assist a person with a limb pathology as well as an exoskeleton.

Additionally, in the context of the invention, the uncertainty shall be understood as a margin of error in a confidence interval which is typically calculated using the standard error of the point estimate, which is a measure of the variability of the sampling distribution of the estimate. The uncertainty is a concept that describes the level of confidence or doubt that one has in the outcome of a particular event or measurement. In the present invention, a model may be used by the state determining means to output the state associated to the motion of the prosthesis, orthosis, or artificial limb. The uncertainty value is linked to the model output regarding the state.

A probability distribution, on the other hand, is a mathematical function that describes the probability of different outcomes in a random process. In a particular embodiment regarding a leg prosthesis, for example, the model used may be trying to approximate the probability function of gait events (defined as series of continuous events or of discrete events). Because the model used may only have data from a sample of amputees, rather than the entire population, there will be some level of uncertainty associated with the estimate of the probability function.

According to a preferred embodiment, the system further comprises a control means configured to control the prosthesis, orthosis, or artificial limb based on the determined state and the obtained uncertainty value.

By knowing the state associated to the motion, and modulating the knowledge of that state with the uncertainty, anticipation of the next motion is improved and elements of the prosthesis, orthosis, or artificial limb may be adjusted to follow up on that anticipation.

Depending on embodiments, the determining of the state associated to the motion may be achieved by determining a current event of the motion or by determining an activity related to the motion. More specifically, according to an exemplary embodiment, the determining of the state associated to the motion comprises determining an activity within a list of activities and/or a motion event within a list of motion events. Additionally or alternatively, instead of a list of discrete activities or a list of discrete motion events, the determining of the activity and/or the current event of the motion may be performed out of a continuum of activities and/or out of a continuum of motion events.

Below, explanations related to the motion in a gait cycle will be given in order to more clearly reach an understanding of events and activities with respect to gait motion. However, the skilled person will understand that these explanations are by way of example only and that the invention can be similarly implemented when considering other types of body motions.

The gait cycle describes the cyclic pattern of motion that occurs while walking. A single cycle of gait starts when the heel of one foot strikes the ground and ends when that same heel touches the ground again. The gait cycle can be divided in two main phases of gait including: the stance phase, and the swing phase. The stance phase is the period of the gait cycle when the foot is on the ground and bearing body weight. More specifically, it can be described as the period between the moment that the heel of the foot touches the ground (heel strike) until the moment that the toe-off occurs. The swing phase is the second phase of gait when the foot is free to move forward. It is described as the period between toe-off and heel strike. Further, each of these phases has subphases.

The stance phase consists of three subphases: the initial loading, the midstance, and the push-off. There are three subphases in the swing phase: the early swing, the mid-swing, and the late swing.

Note that the definitions above relative to the gait cycle while walking is by way of example only. The skilled person will understand that different gait patterns may be defined depending on different types of activities such as climbing or going down stairs, biking, going uphill or downhill, etc. Note also that some gait patterns do not present a time periodicity.

Therefore, in an embodiment, forces analysis during motion, *e.g.* during the gait motion, may be performed based on the sensed data, and determining the current event of the motion may comprise determining a main phase of the motion and/or determining a subphase of the motion within the list of main phases and/or subphases of the motion.

Additionally or alternatively, motion activity analysis, *e.g.* gait activity analysis, may be performed based on the sensed data, and determining the activity related to the motion may be based on the motion activity analysis.

Here, by activity, it is meant the type of motion implemented thanks to or via the prosthesis, orthosis, or artificial limb of the user. An activity may be defined based on a trajectory of motion, *e.g.* a gait pattern, a time duration of motion, and/or on various data sensed during the execution of the motion. An activity may also be associated with an activity level, *i.e.* how active a user is. An activity level may be determined *e.g.* based on the sensed motion. For example, in relation with the gait motion, the list of activities includes any one of the following: standing, jumping, walking (at different speed levels), running (at different speed levels), sitting, driving, using stairs, going at an incline (up or down), biking, laying down, etc.

Preferably, the state determining means uses one model per activity.

According to an exemplary embodiment, the state determining means is further configured to determine a plurality of state candidates, each of the plurality of state candidates being determined using a different state determining model. The uncertainty value is obtained based on the plurality of state candidates.

Instead of using only one model, the state determining means may use a plurality of models to determine the plurality of state candidates. Each of the plurality of models may be trained differently and may have different performances and precisions depending on a given state. By taking into account the plurality of state candidates, a more accurate prediction of the state associated with the motion can be reached.

According to a preferred embodiment, the state determining means is integrated or attached to the prosthesis, orthosis, or artificial limb and/or included in a wearable or mobile device.

According to a particular embodiment, the at least one sensing means is integrated or attached to the prosthesis, orthosis, or artificial limb and/or included in a wearable or mobile device.

According to an exemplary embodiment, the state determining means is further configured to estimate a motion quality value of the motion performed by the user, said motion quality value being based on optimal motion mechanical criteria. In this manner, abnormal or sub-optimal body motion may be detected, which could lead to early detection of pathologies.

Still taking the example of the gait cycle, it is to note that walking requires the healthy functioning of several body systems including the musculoskeletal, nervous, cardiovascular and respiratory systems. A loss of healthy gait function can lead to falls, injuries, loss of movement and personal freedom, and a significantly reduced quality of life. Therefore, estimating the motion quality value is valuable in improving life of the user wearing or equipped with the prosthesis, orthosis, or artificial limb.

Sensed data related to the gait motion may be used for gait analysis in order to assess spatial, time, and temporal variables of the gait motion. These variables may include the limb movement and positions, joint angles, trajectories, velocities, generated force and muscle activity of particular body segments during the various phases of the gait cycle. Then, kinematic and biomechanical equations can be calculated to determine variations from known norms and establish a gait pattern of the user wearing the prosthesis or orthosis, or equipped with the artificial limb. It is known that each individual has a characteristic gait pattern. It can depend on a number of individual variables such as age, height, weight, sex, walking speed, strength, flexibility, type of surgery, shape of the amputation, and aerobic conditioning. The gait patterns can be assessed by conducting the gait analysis.

The alterations in normal gait can be caused by different deformities, injuries, weakness, disease, or pain in any part of the body. For example, a loss of dorsiflexion could suggest nerve root compression, peroneal nerve compression, stroke or a neurological condition such as multiple sclerosis. A departure from the normal gait may be detected by estimating the motion quality value of the motion performed by the subject, a high motion quality value being associated to a normal gait.

By sensing data related to the motion performed by the user involving the prosthesis, orthosis, or artificial limb worn or equipped by said user, relevant data can be gathered and can be used for improving the use of the prosthesis, orthosis, or artificial limb. The sensed data may be raw data which was directly obtained, *e.g.* by the at least one sensing means, pre-processed data based on the directly obtained data, or post-processed data.

Another type of data related to the motion and involving the prosthesis, orthosis, or artificial limb may be data such as logs of control data used during use of the prosthesis, orthosis, or artificial limb, inputs to the processor of the prosthesis, orthosis, or artificial limb, physical characteristics of different elements composing the prosthesis, orthosis, or artificial limb, algorithms used for the operation of the prosthesis, orthosis, or artificial limb, software data for the processor, user data (feedback) related to the felt performance, and structure data of the inputs to the model.

Additional sensed data may originate from sensing means that may be comprised in the prosthesis, orthosis, or artificial limb, said sensing means pertaining to the inner working of the prosthesis, orthosis, or artificial limb. Environmental data related to the environment of the prosthesis, orthosis, or artificial limb may be sensed data of sensing means comprised in the prosthesis, orthosis, or artificial limb, said sensing means pertaining to the surroundings of the prosthesis, orthosis, or artificial limb. The data related to the environment may relate to parts of the surroundings in which the user wearing the orthosis, prosthesis, or user equipped with the artificial limb is present or may relate to parts of the wearer or subject (*i.e.* user), such as data about a part of the body of the wearer or subject. In an embodiment, data related to the environment may be pertaining to a signal from a neural sensor implanted in the wearer of the prosthesis, orthosis, or artificial limb. Such signal(s) may include motion commands for the prosthesis, orthosis, or artificial limb.

The sensed data may be stored in a data storing means connected to the prosthesis, orthosis, or artificial limb.

At least part of the sensed data may be then transferred to a remote server. Alternatively or additionally, pre-processed data and/or post-processed data based on the sensed data may be transferred to the remote server. The data may be processed by a processing means of the prosthesis, orthosis, or artificial limb and/or a processing means of a communication module connected to the prosthesis, orthosis, or artificial limb. By transferring data to the remote server, data can be accumulated at the level of the remote server and be subject to data analysis for various purposes.

The data may be transferred to the remote server using the communication module. The communication module may be part of the prosthesis, orthosis, or artificial limb, may be an independent unit, and/or may be part of another device separate from the prosthesis, orthosis, or artificial limb. The communication module may interface with the data storing means connected to the prosthesis, orthosis, or artificial limb. The interfacing may be achieved wirelessly or in a wired manner. The communication module and the remote server may communicate wirelessly or in a wired manner.

It is to be noted that the processing of data stored in the data storing means may be performed directly after the data is stored or at an ulterior time. Also, the transferring of data to the remote server may be performed directly after the data is stored, after the processing of the stored data, or at an ulterior time.

In the context of the invention the term "remote server" can refer to a single server or a group of servers, *e.g.* a distributed set of servers, such as an edge cloud.

According to a preferred embodiment, the method further comprises receiving, using the communication module, configuration and/or behavioral data from the remote server. The configuration and/or behavioral data may be obtained based on the transferred data.

In this manner, configuration and/or behavioral data can be transferred to the prosthesis, orthosis, or artificial limb without the need for a personal intervention by the operator. The configuration and/or behavioral data influences the way the prosthesis, orthosis, or artificial limb operates and/or behaves. Thus, the configuration and/or behavioral data may be optimized for each individual based on the data transferred to the remote server. This may further improve the time-efficiency of the maintenance or servicing. The configuration data may comprise control data for the operation of the prosthesis, orthosis, or artificial limb. Typically, the configuration data may control the internal operation of the prosthesis, orthosis, or artificial limb, and the configuration data may be updated by the remote server based on internal information, such as errors, logs, etc. received from the prosthesis, orthosis, or artificial limb. The behavioral data may comprise parameters used by the processor during operation of the prosthesis, orthosis, or artificial limb. Typically, the behavioral data will determine the external operation of the prosthesis or orthosis, *i.e.* how the prosthesis, orthosis, or artificial limb behaves with respect to the actions or activity the user is performing or in response to environmental properties (*e.g.* type of grounds, stairs, slopes, information about the person or about a body part of the person wearing the prosthesis or orthosis, or equipped with the artificial limb, etc.). The behavioral data may comprise software code, such as the controlling model for controlling the operation of a force, resistance or motion generating device for influencing the mechanical behavior and/or movement of a component of the orthosis, prosthesis, or artificial limb.

By being able to alter configuration and/or behavioral data remotely, the configuration and/or behavioral data can be updated in an improved manner.

According to an exemplary embodiment, the orthosis, prosthesis, or artificial limb comprises a force, resistance or motion generating device, such as an actuator or a damper, for influencing the mechanical behavior and/or movement of an element of the orthosis, prosthesis, or artificial limb, and a control module (or control means) configured to control the force, resistance or motion generating device according to the control agent. The configuration and/or behavioral data received from the remote server may be used to modify the controlling agent.

In this way, the operation of the prosthesis, orthosis, or artificial limb may be altered remotely by using data transfer. The actuator may be pneumatic, piezo-electric, electric, hydraulic, magnetic, or mechanical. In some embodiments the altering may be done "online", *e.g.* as soon as the data is available and in other embodiments the altering may be done "offline", *e.g.* the data may be stored first and used later. Also, combinations are possible, *e.g.* some alterations could be done "online" and others "offline". The damper may be active or passive. In a prosthesis, orthosis, or artificial limb comprising a control module and a force, resistance or motion generating device configured for influencing the mechanical behavior and/or movement of elements of the prosthesis, orthosis, or artificial limb, different parameters may be considered which serve to quantify said mechanical impact.

The control agent may receive one or more inputs and may use one or more weight parameters to generate one or more outputs based on the one or more inputs. The control agent may define a control policy, *e.g.* as known in the technical domain of artificial intelligence. For example, the one or more inputs may comprise outputs of one or more sensing means, and/or processed data based on the outputs of one or more sensing means, and/or other data inputs.

The configuration and/or behavioral data sent to the communication module may include the parameters mentioned above related to the mechanical behavior and/or movement of an element of the orthosis, prosthesis, or artificial limb. However, the skilled person will understand that the configuration and/or behavior data is not limited to those parameters and may comprise data relevant to electronic components, or electronic elements included in components involved in the operation of the prosthesis, orthosis, or artificial limb such as for battery management, thermal management, data storage management, clock, etc. In an embodiment, the configuration and/or behavioral data may define one or more new sources of inputs for the control agent, or the configuration and/or behavioral data may include a new control agent itself.

According to a preferred embodiment, the at least one sensing means comprises any one or any combination of: a neural sensor, an angle-sensing means, an accelerometer, a gyroscope, a Hall sensor, a force sensor, a magnetometer, a pressure sensor, a torque sensor, a temperature sensor, an energy metering means, a current sensor, a voltage sensor, a humidity sensor, a sonar sensor, an EMG sensor, a barometric sensor, a grid of pressure sensor, an EEG sensor, a RFID sensor, a geo-localization sensor.

In this manner, various types of data may be sensed and collected relative to the operation and/or environment of the prosthesis, orthosis, or artificial limb. Depending on the sensor(s) chosen, different algorithm models of the state determining means using the sensed data may be developed in order to infer the state of the prosthesis, orthosis, or artificial limb.

According to an exemplary embodiment, the at least one sensing means comprises multiple sensors. One or more sensors of said multiple sensors are selected to provide an input of the control agent based on the configuration and/or behavioral data.

In this manner, the most appropriate sensor or set of sensors may be selected in order to regulate in an improved manner the mechanical behavior and/or movements of the prosthesis, orthosis, or artificial limb via the force, resistance or motion generating device. Thus, depending on the configuration and/or behavioral data, different sets of one or more sensors may be used as an input of the controlling model. Additionally or alternatively, different sets of one or more sensors may be used as an input of the control agent depending on environmental data. Also, sensed data of different sets of one or more sensors may be used as an input of the state determining means depending on environmental data.

The skilled person will understand that the hereinabove described technical considerations and advantages related to embodiments of a system comprising a prosthesis, orthosis, or artificial limb also apply to the below described corresponding controlling method embodiments, *mutatis mutandis*.

According to a second aspect of the invention, there is provided a method for controlling a prosthesis, orthosis, or artificial limb, in a system comprising the prosthesis, orthosis, or artificial limb, and at least one sensing means, and a state determining means. The method comprises the steps of, repeatedly:
- obtaining sensed data, from the at least one sensing means, related to a motion performed by a user involving the prosthesis, orthosis, or artificial limb worn by said user;
- determining, by the state determining means, a state associated to the motion based on the sensed data;
- obtaining, by the state determining means, an uncertainty value representative for a level of uncertainty that the user is in the determined state.

The skilled person will understand that the hereinabove described technical considerations and advantages related to embodiments of a method for controlling a prosthesis, orthosis, or artificial limb also apply to the below described corresponding control agent self-development method embodiments, *mutatis mutandis*.

According to a third aspect of the invention, there is provided a method for self-development of a control agent for a prosthesis, orthosis, or artificial limb. The control agent is configured for being used by a control means of the prosthesis, orthosis, or artificial limb in controlling said prosthesis, orthosis, or artificial limb. The method comprises the steps of:
- obtaining a motion model of the prosthesis, orthosis, or artificial limb, said motion model defining mechanical relationships between elements of the prosthesis, orthosis, or artificial limb;
- obtaining a training response model of the prosthesis, orthosis, or artificial limb, said training response model defining a predefined motion response of the prosthesis, orthosis, or artificial limb;
- determining a current motion state of the prosthesis, orthosis, or artificial limb; and
repeatedly:
- determining, by the control agent, a candidate mechanical action based on the current motion state, said mechanical action related to the elements of the prosthesis, orthosis, or artificial limb;
- outputting, by the motion model, a next candidate motion state based on the current motion state and the candidate mechanical action;
- outputting, by the training response model, a next predefined motion state based on the current motion state;
- obtaining a reward value based on the next candidate motion state and the next predefined motion state, said reward value being associated to the current motion state and the candidate mechanical action;
- setting the next candidate motion state as the current motion state.

Here, the motion model is a virtual model designed to reproduce the prosthesis, orthosis, or artificial limb mechanically, from a sensor point of view, and also considering the controlling capabilities of said prosthesis, orthosis, or artificial limb. In an embodiment, the motion model may be implemented with a neural network.

Using this virtual reproduction of the prosthesis, orthosis, or artificial limb, the predefined motion response would be the desired mechanical outputs of the prosthesis, orthosis, or artificial limb using sensed data as the inputs. The desired mechanical outputs are based on a plurality of natural equivalent responses of the user wearing or equipped with the prosthesis, orthosis, or artificial limb. The ensemble of natural equivalent responses from the subject wearing or equipped with the prosthesis, orthosis, or artificial limb are gathered in the training response model. The training response model may be acquired based on, for example, gait analysis of a healthy limb of the user, using an able-body subject, or using kinematic simulations based on physical characteristics of the user's body.

Complex relationships may be developed between sensed data as inputs and desired mechanical outputs. The ensemble of these complex relationships are gathered in the control agent, which may also be a model. Starting from an initial control agent, two aims are pursued for its development. The first aim is to rate its performances by comparing the plurality of candidate mechanical actions through multiple iterations and their results with respect to the training response model using reward values. The second aim is to use the reward values which have been accumulated through the iterations so that the plurality of candidate motion states obtained from the plurality of candidate mechanical actions is closer to corresponding portions of the training response models.

To achieve the first aim, starting from a current motion state, the control agent will be left to proceed with determining the candidate mechanical action that should follow in order to reach the next logical motion state from the viewpoint of the control agent. The next logical motion state is called here the next candidate motion state. This next candidate motion state is obtained by taking into account the candidate mechanical action and how it affects the prosthesis, orthosis, or artificial limb thanks to the motion model. However, this next logical motion state may differ from the development of motion states as defined in the training response model. Comparing both, the reward value will be attributed to the couple constituted by the current motion state and the candidate mechanical action. The steps above will then be repeated from the new motion state reached, thereby describing a series of motion states coupled with mechanical actions and associated with reward values.

In this manner, a strategy is developed which will help in guiding the development of the control agent so that a series of mechanical actions lead to a series of motion states mimicking a corresponding series of motion states from the training response model.

More specifically, in order to check whether the modifications of the control agent are going in the right direction, a reward-based strategy is used. This strategy is a way to train an AI (Artificial Intelligence) to match a given expectation which is called reinforcement learning. The reward value may be provided by a simple algorithm that can grade any output. Here, the grade, *i.e.* the reward value, may be calculated by comparing the difference between the training response model and series of motion states obtained using the control agent. The reward values obtained iteration after iteration may be inputted to a cumulative reward function over time. A score derived from the cumulative reward function may be calculated, and if the score reaches above a predetermined threshold, the control agent is modified to reinforce the series of candidate mechanical actions determined during the iterations; otherwise the previous version of the modified control agent is employed. Depending on embodiments, the score may correspond to the last value of the cumulative reward function, or to the integral value of the cumulative reward function. Iterations of modification and attribution of rewards values are repeated until motions corresponding to the training response model can be obtained using the control agent and the motion model.

Additionally or alternatively, multiple control agents may be developed and compared based on their respective cumulative reward functions. And if the cumulative reward function associated with one of the multiple control agents dominates the ones of the other control agents (based on a comparison of the scores derived from the multiple cumulative reward functions), that control agent may be modified to reinforce the series of candidate mechanical actions determined during the iterations.

In this manner, the control agent for the prosthesis, orthosis, or artificial limb is developed using AI to more closely correspond to a natural body motion. Depending on embodiments, the control agent may be developed to correspond to a single activity of the user, or can be developed to be adequate in being used for a continuum of motion states and activities.

According to an exemplary embodiment, the method further comprises the steps of:
- upon reaching a predetermined condition, determining a cumulative reward function based on the obtained one or more reward values;
- modifying the control agent based on a score derived from the cumulative reward function.

The iterations may continue until the predetermined condition is reached. In an embodiment, the predetermined condition may be a predetermined number of iterations. In another embodiment, the predetermined condition may be a convergence in the cumulative reward function based on the one or more the reward values in function of the number of iterations. In yet another embodiment, the predetermined condition may be obtaining rewards values below a predetermined threshold in succession.

Preferably, the predetermined condition includes: a predetermined number of iterations of the repeated steps, or a predetermined threshold value for a convergence characteristic of the cumulative reward function.

Depending on the cumulative reward function, the control agent may then be modified. If the score derived from the cumulative reward function is below a first score threshold, the first of the candidate mechanical action may be marked as a "dead-end" and the control agent may be made to start again going through multiple iterations from the same initial current motion state. If the score derived from the cumulative reward function is above the first score threshold or a second score threshold, then some parameters of the control agent may be reinforced so that similar mechanical actions as the candidate mechanical actions are employed when using the control agent with the control means of the prosthesis, orthosis, or artificial limb.

Additionally, the modifying of the control agent may comprise modifying a reward-grading algorithm used to obtain the reward values during the one or more iterations of the repeated steps. In this manner, the reinforcement learning may be biased using the reward-grading algorithm to favor some candidate mechanical actions with respect to others.

According to a preferred embodiment, the modifying of the control agent is further based on a user reward input from a user wearing or equipped with the prosthesis, orthosis, or artificial limb, said user reward input being associated to a performance of the modified control agent used by the control means of the prosthesis, orthosis, or artificial limb in controlling the worn or equipped prosthesis, orthosis, or artificial limb.

In this way, an additional source of input can be taken in consideration when developing the control agent. To obtain this input, the control agent in-development may be implemented in a real prosthesis, orthosis, or artificial limb corresponding to the virtually reproduced one and a rating may be given by the user based on the use of the prosthesis, orthosis, or artificial limb controlled using the control agent in-development. The user reward input is calculated based on the rating and used to complement, or instead of, the calculation performed by the reward-grading algorithm in order to obtain the reward value.

According to an exemplary embodiment, the modifying of the control agent is further based on an extracted reward input being associated to a performance of the modified control agent used by the control means of the prosthesis, orthosis, or artificial limb in controlling the worn or equipped prosthesis, orthosis, or artificial limb. The extracted reward input may be based on measures extracted from one or more evaluating sensing means during use of the prosthesis, orthosis, or artificial limb by the user. The one or more evaluating sensing means may be sensing means comprised by the prosthesis, orthosis, or artificial limb, or may be sensing means external to the prosthesis, orthosis, or artificial limb. The measures extracted may be related to one or more of biomechanical characteristics, physical performance characteristics, physiological characteristics, and/or psychological characteristics. The psychological characteristics may be retrieved based on questionnaires, ratings of perceived exertion, and/or visual analogue scales.

In the specific case of a leg-related prosthesis, orthosis, or artificial limb, the measures extracted may be related, for example, to one or more of the following: symmetry, cadence, center of motion, center of pressure, double stance time, ground reaction forces, power - ankle, power - hip, power - knee, range of motion - ankle, range of motion - hip, range of motion - knee, range of motion - trunk, range of motion - upper limb, single stance time, stance time, step length, step width, stride length, stride time, swing time, torque - ankle, torque - hip, torque - knee, distance, duration hill assessment index, reaction time, speed, stair assessment index, breathing frequency, carbon dioxide production, electro-encephalography, electro-myography, heart rate, metabolic rate, oxygen consumption, oxygen cost, respiratory exchange ratio, skin conductance, ventilatory equivalent.

According to a particular embodiment, the current motion state of the prosthesis, orthosis, or artificial limb is defined based on one or more sensed data from a list of available sensed data during use of the prosthesis, orthosis, or artificial limb.

According to an exemplary embodiment, the motion model, the training response model, the control agent, and the trained response model are associated to an activity performed by the user of the prosthesis, orthosis, or artificial limb.

The skilled person will understand that the hereinabove described technical considerations and advantages related to gait transitions determining method embodiments during use of a prosthesis, orthosis, or artificial limb, controlling method embodiments for controlling a prosthesis, orthosis, or artificial limb, and self-development method of a controlling model embodiments also apply to the below described corresponding development method embodiments, *mutatis mutandis*.

According to a fourth aspect of the invention, there is provided a method for development of a plurality of controlling models for a prosthesis, orthosis, or artificial limb. The method comprises the steps of:
- obtaining a motion model of the prosthesis, orthosis, or artificial limb, said motion model defining mechanical relationships between elements of the prosthesis, orthosis, or artificial limb;
- obtaining a plurality of training response models of the prosthesis, orthosis, or artificial limb, each of said plurality of training response models defining a predefined motion response of the prosthesis, orthosis, or artificial limb and being associated to a biomechanically different user wearing or equipped with the prosthesis, orthosis, or artificial limb;
- obtaining an initial plurality of control agents, preferably similar control agents, each of the initial plurality of control agents being associated to a different training response model of the plurality of training response models, and configured for being used by a control means of the prosthesis, orthosis, or artificial limb in controlling the worn or equipped prosthesis, orthosis, or artificial limb;
- selecting a control agent among the initial plurality of control agents, and defining the selected control agent as the favored control agent; and
repeatedly:
- selecting a challenger control agent among the plurality of control agents, the challenger control agent being different from the favored control agent, and preferably not being selected in a previous iteration;
- obtaining a user input indicative of a user preference among the challenger control agent and the favored control agent relative to a performance of the challenger and favored control agents, respectively, during use by the control means of the prosthesis, orthosis, or artificial limb in controlling the worn or equipped prosthesis, orthosis, or artificial limb;
- based on the user input, selecting a new favored control agent between the previous favored control agent and the challenger control agent.

In this manner, the plurality of control agents can be rated in a competition against each other so that the one or more control agents suited most to the user, which is rating them according to preference, are selected over one or more iterations of the repeated steps above.

The skilled person will understand that the initial plurality of control agents may be obtained by any known method. Alternatively or additionally, the initial plurality of control agents may be obtained by the self-development method described prior.

By using the plurality of control agents, each one associated to different training response models of the plurality of training response models, the competition will allow determining which one or more of the initial plurality of control agents is closer to a desired tailored control agent from the user performing the rating. In other words, one or more of the better starting points for the further development of the desired tailored control agent is selected by the above method.

According to a preferred embodiment, the method further comprises the step of, upon reaching a
predetermined condition, identifying a final favored control agent
Preferably, the predetermined condition includes a predetermined number of selections of the same favored control agent during iterations of the repeated steps.

According to an exemplary embodiment, the method further comprises the steps of:
- selecting the final favored control agent as a first control agent; and
the method further comprises the steps of, repeatedly:
- generating a second control agent based on the first control agent, preferably by replicating the first control agent;
- training the second control agent based on the motion model of the prosthesis, orthosis, or artificial limb and the training response model associated with the first control agent, said training being preferably performed during a predetermined number of epochs;
- obtaining a further user input indicative of a further user preference among the first control agent and the second control agent relative to a performance of the first and the second control agents, respectively, during use by the control means of the prosthesis, orthosis, or artificial limb in controlling the worn or equipped prosthesis, orthosis, or artificial limb;
- based on the further user input, selecting a new first control agent between the previous first control agent and the second control agent.

In this way, the final favored control agent may be further developed in an iterative manner by, generation after generation of training, obtaining using user input the new first control agent which is closer to the desired tailored control agent.

According to a preferred embodiment, the obtaining of the initial plurality of control agents is based on one or more sensed data from a list of available sensed data during use of the prosthesis, orthosis, or artificial limb.

The skilled person will understand that the hereinabove described technical considerations and advantages related to development method of a plurality of controlling models embodiments, and self-development method of a controlling model embodiments also apply to the below described corresponding system embodiments, *mutatis mutandis*.

According to a fifth aspect, there is provided a system comprising a prosthesis, orthosis, or artificial limb. The system further comprises: at least one sensing means configured to sense data related to a motion performed by a user involving the prosthesis, orthosis, or artificial limb worn or equipped by said user; and a control means configured to use a control agent to control an element of the prosthesis, orthosis, or artificial limb. The control agent used is the modified control agent self-developed according to the method of the third aspect or a selected new favored control agent developed according to the method of the fourth aspect.

According to a further aspect of the invention, there is provided a computer program comprising computer-executable instructions to perform any of the above described methods, when the program is run on a computer or, according to any one of the steps of any one of the embodiments disclosed above.

According to a further aspect of the invention, there is provided a computer device or other hardware device programmed to perform one or more steps of any one of the embodiments of the methods disclosed above. According to another aspect there is provided a data storage device encoding a program in machine-readable and machine-executable form to perform one or more steps of any one of the embodiments of the various methods disclosed above.

Additional features and aspects of control agent development method embodiments, system embodiments, and computer program embodiments are defined by the following set of clauses:
1. A method for self-development of a control agent for a prosthesis, orthosis, or artificial limb, said control agent configured for being used by a control means of the prosthesis, orthosis, or artificial limb in controlling said prosthesis, orthosis, or artificial limb, wherein the method comprises the steps of:
   - obtaining a motion model of the prosthesis, orthosis, or artificial limb, said motion model defining mechanical relationships between elements of the prosthesis, orthosis, or artificial limb;
   - obtaining a training response model of the prosthesis, orthosis, or artificial limb, said training response model defining a predefined motion response of the prosthesis, orthosis, or artificial limb;
   - determining a current motion state of the prosthesis, orthosis, or artificial limb;
   wherein the method further comprises the steps of, repeatedly:
   - determining, by the control agent, a candidate mechanical action based on the current motion state, said mechanical action related to the elements of the prosthesis, orthosis, or artificial limb;
   - outputting, by the motion model, a next candidate motion state based on the current motion state and the candidate mechanical action;
   - outputting, by the training response model, a next predefined motion state based on the current motion state;
   - obtaining a reward value based on the next candidate motion state and the next predefined motion state, said reward value being associated to the current motion state and the candidate mechanical action;
   - setting the next candidate motion state as the current motion state.
2. The method of clause 1, wherein the method further comprises the steps of:
   - upon reaching a predetermined condition, determining a cumulative reward function based on the obtained one or more reward values;
   - modifying the control agent based on a score derived from the cumulative reward function.
3. The method of clause 2, wherein the modifying of the control agent is further based on a user reward input from a user wearing or equipped with the prosthesis, orthosis, or artificial limb, said user reward input being associated to a performance of the modified control agent used by the control means of the prosthesis, orthosis, or artificial limb in controlling the worn or equipped prosthesis, orthosis, or artificial limb.
4. The method of any one of clauses 1-3, wherein the current motion state of the prosthesis, orthosis, or artificial limb is defined based on one or more sensed data from a list of available sensed data during use of the prosthesis, orthosis, or artificial limb.
5. The method of clause 4, wherein the list of available sensed data comprises data sensed by any one or more of the following sensing means: a neural sensor, an angle-sensing means, an accelerometer, a gyroscope, a Hall sensor, a force sensor, a magnetometer, a pressure sensor, a torque sensor, a temperature sensor, an energy-metering means, a current sensor, a voltage sensor, a humidity sensor, a sonar sensor, an EMG sensor, a barometric sensor, a grid of pressure sensors, an EEG sensor, a RFID sensor, a geo-localization sensor.
6. The method of clause 2 or 3, wherein the predetermined condition includes: a predetermined number of iterations of the repeated steps, or a predetermined threshold value for a convergence characteristic of the cumulative reward function.
7. The method of any one of clause s 2-6, wherein the modifying of the control agent comprises modifying a reward-grading algorithm used in obtaining the reward value.
8. The method of any one of clauses 1-7, wherein the elements of the prosthesis, orthosis, or artificial limb includes an actuator, and wherein the determining of the candidate mechanical action comprises determining a mechanical action of the actuator.
9. The method of any one of clauses 1-7, wherein the training response model is associated to the user wearing or equipped with the prosthesis, orthosis, or artificial limb.
10. The method of any one of clauses 1-9, wherein the training response model is associated to an activity within a list of activities, and wherein the list of activities includes any one of the following: walking, running, sitting, driving, using stairs, going at an incline, biking, laying down, standing, jumping.
11. A method for development of a favored control agent for a prosthesis, orthosis, or artificial limb, comprising the steps of:
   - obtaining a motion model of the prosthesis, orthosis, or artificial limb, said motion model defining mechanical relationships between elements of the prosthesis, orthosis, or artificial limb;
   - obtaining a plurality of training response models of the prosthesis, orthosis, or artificial limb, each of said plurality of training response models defining a predefined motion response of the prosthesis, orthosis, or artificial limb and being associated to a biomechanically different user wearing or equipped with the prosthesis, orthosis, or artificial limb;
   - obtaining an initial plurality of control agents, preferably similar control agents, each of the initial plurality of control agents being associated to a different training response model of the plurality of training response models, and configured for being used by a control means of the prosthesis, orthosis, or artificial limb in controlling the worn or equipped prosthesis, orthosis, or artificial limb;
   - selecting a control agent among the initial plurality of control agents, and defining the selected control agent as the favored control agent;
   wherein the method further comprises the steps of, repeatedly:
   - selecting a challenger control agent among the plurality of control agents, the challenger control agent being different from the favored control agent, and preferably not being selected in a previous iteration;
   - obtaining a user input indicative of a user preference among the challenger control agent and the favored control agent relative to a performance of the challenger and favored control agents, respectively, during use by the control means of the prosthesis, orthosis, or artificial limb in controlling the worn or equipped prosthesis, orthosis, or artificial limb;
   - based on the user input, selecting a new favored control agent between the previous favored control agent and the challenger control agent.
12. The method according to clause 11, wherein the method further comprises the step of:
   - upon reaching a predetermined condition, identifying a final favored control agent.
13. The method according to clause 12, wherein the method further comprises the steps of:
   - selecting the final favored control agent as a first control agent; and
   wherein the method further comprises the steps of, repeatedly:
   - generating a second control agent based on the first control agent, preferably by replicating the first control agent;
   - training the second control agent based on the motion model of the prosthesis, orthosis, or artificial limb and the training response model associated with the first control agent, said training being preferably performed during a predetermined number of epochs;
   - obtaining a further user input indicative of a further user preference among the first control agent and the second control agent relative to a performance of the first and the second control agents, respectively, during use by the control means of the prosthesis, orthosis, or artificial limb in controlling the worn or equipped prosthesis, orthosis, or artificial limb;
   - based on the further user input, selecting a new first control agent between the previous first control agent and the second control agent.
14. The method of any one of clauses 11-13, wherein the obtaining of the initial plurality of control agents is based on one or more sensed data from a list of available sensed data during use of the prosthesis, orthosis, or artificial limb.
15. The method of clause 14, wherein the list of available sensed data comprises data sensed by any one or more of the following sensing means: a neural sensor, an angle-sensing means, an accelerometer, a gyroscope, a Hall sensor, a force sensor, a magnetometer, a pressure sensor, a torque sensor, a temperature sensor, an energy-metering means, a current sensor, a voltage sensor, a humidity sensor, a sonar sensor, an EMG sensor, a barometric sensor, a grid of pressure sensors, an EEG sensor, a RFID sensor, a geo-localization sensor.
16. The method of any one of clauses 12-15, wherein the predetermined condition includes a predetermined number of selections of the same favored control agent during iterations of the repeated steps.
17. The method of any one of clauses 11-16, wherein the plurality of training response models is associated to an activity within a list of activities, and wherein the list of activities includes any one of the following: walking, running, sitting, driving, using stairs, going at an incline, biking, laying down, standing, jumping.
18. A system comprising a prosthesis, orthosis, or artificial limb, said system further comprising:
   - at least one sensing means configured to sense data related to a motion performed by a user involving the prosthesis, orthosis, or artificial limb worn or equipped by said user;
   - a control means configured to use the modified control agent self-developed according to the method of clause 2 or the favored control agent developed according to the method of clause 11 in controlling elements of the prosthesis, orthosis, or artificial limb.
19. The system of clause 18, further comprising at least one user interface device configured to receive a user input.
20. The system of clause 18 or 19, wherein the control means is integrated or attached to the prosthesis, orthosis, or artificial limb, and/or included in a wearable device or mobile device.
21. The system of any one of clauses 18-20, wherein the at least one sensing means is integrated or attached to the prosthesis, orthosis, or artificial limb, and/or included in the wearable device or the mobile device.
22. A computer program comprising instructions which, when the program is executed by a computer, causes the computer to carry out the steps of the method according to any one of clauses 1-10, or causes the computer to carry out the steps of the method according to any one of clauses 11-17.

### BRIEF DESCRIPTION OF THE DRAWINGS

This and other aspects of the present invention will now be described in more detail, with reference to the appended drawings showing a currently preferred embodiment. Like numbers refer to like features throughout the drawings.
Figure 1 shows a schematic view of an exemplary embodiment of a system comprising a prosthesis, orthosis, or artificial limb communicating with a secondary device;
Figure 2 depicts a flow chart of an exemplary embodiment of a method for controlling a prosthesis, orthosis, or artificial limb, in a system comprising the prosthesis, orthosis, or artificial limb;
Figure 3 depicts a flow chart of an exemplary embodiment of a method for self-development of a control agent for a prosthesis, orthosis, or artificial limb;
Figure 4 depicts a flow chart of an exemplary embodiment of a method for development of a favored control agent for a prosthesis, orthosis, or artificial limb;
Figure 5 depicts a flow chart of an exemplary embodiment of a method for further development of a final favored control agent for a prosthesis, orthosis, or artificial limb.

### DETAILED DESCRIPTION OF EMBODIMENTS

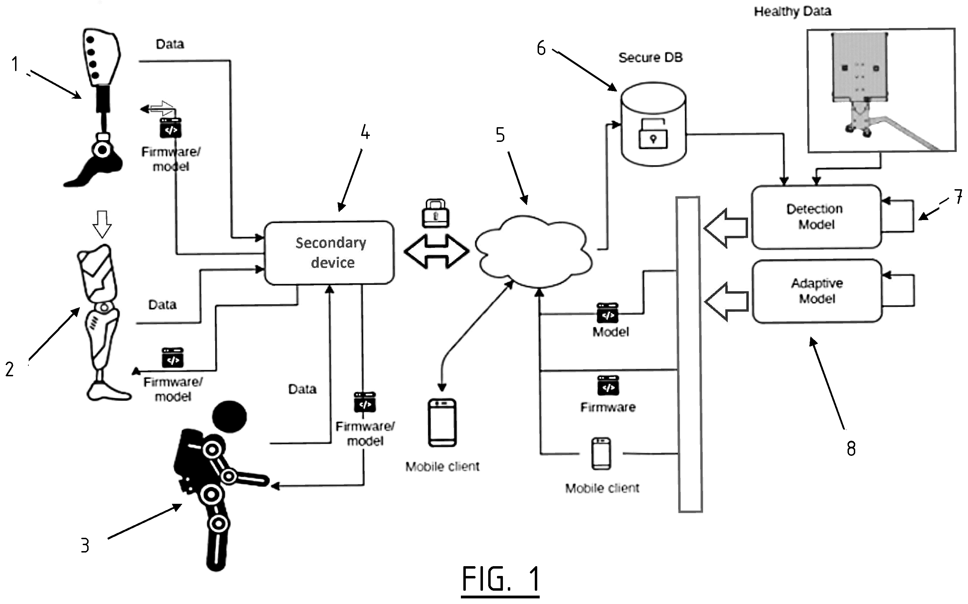

Figure 1 shows a schematic view of an exemplary embodiment of a system comprising a prosthesis, orthosis, or artificial limb communicating with a secondary device according to the present invention. The prosthesis 1, orthosis 2 may be configured for functionally assisting, enhancing, and/or replacing a limb of a human or animal subject or for augmenting a body or a part of a body of a human or animal subject. The artificial limb 3 may be configured for functionally acting as a limb of a humanoid or animal-inspired robot. The term "orthosis" is used to refer both to a device used to assist a person with a limb pathology, as well as to a device that augments the performance of an able-bodied wearer. Thus, when referring to an orthosis, this could be an orthosis used to assist a person with a limb pathology as well as an exoskeleton. The prosthesis 1, orthosis 2, or artificial limb 3 includes at least one sensing means, and a state determining means. The at least one sensing means is configured to sense data related to a motion performed by a user involving the prosthesis, orthosis, or artificial limb worn or equipped by said user. The state determining means is configured to repeatedly perform the following steps: determine a state associated to the motion based on the sensed data, said sensed data being obtained from the at least one sensing means; and obtain an uncertainty value representative for a level of uncertainty that the user is in the determined state.

The prosthesis 1, orthosis 2, or artificial limb 3 may include a data storing means and a processing means. The state determining means may be included in the processing means. A communication module connected to the data storing means of the prosthesis 1, orthosis 2, or artificial limb 3 may communicate with a secondary device 4. Depending on embodiments, one or more of the prosthesis 1, orthosis 2, or artificial limb 3 may be in communication with the secondary device 4. The secondary device 4 may be a remote server or a mobile device.

The prosthesis 1, orthosis 2, or artificial limb 3 may comprise a force, resistance or motion generating device such as actuators and/or dampers for influencing the mechanical behavior and/or movement of an element of the prosthesis 1, orthosis, or artificial limb. The prosthesis 1, orthosis 2, or artificial limb 3 may also comprise a control means. In the prosthesis 1, orthosis 2, or artificial limb 3 comprising the control means, said control means may be configured for controlling such force, resistance or motion generating device, and different parameters may be considered to achieve such controlling. For example, parameters may be used in feedback loops as weights for different considered inputs, such as sensed data. A force, resistance or motion generating device, such as an actuator and/or a damper may then be actively or passively controlled based on the feedback loops.

The data storing means may be configured for storing data related to an operation of the prosthesis 1, orthosis 2, or artificial limb 3 and/or to an environment of the prosthesis 1, orthosis 2, or artificial limb 3. The communication module may comprise a communication interface configured for transferring at least part of the stored data and/or processed data based thereon, to the secondary device 4. The secondary device 4 may itself be connected, directly or indirectly, to a cloud network 5, preferably by encrypted connection. The at least part of the stored data and/or processed data transferred to the secondary device 4 may be stored in a secure database 6 for further processing.

Different models 7, 8 may be determined and/or developed using data from the secure database 6. Other types of data, such as firmware data or mobile client data may also be obtained based on the data from the secure database 6. For example, configuration and/or behavioral data based on the data from the secure database 6 may be obtained and sent back to the secondary device 4 via the cloud network 5. Then, the communication module of the prosthesis 1, orthosis 2, or artificial limb 3 may be configured to receive the configuration and/or behavioral data from the secondary device 4.

The prosthesis 1, orthosis 2, or artificial limb 3 may comprises a force, resistance or motion generating device, such as an actuator or a damper, for influencing the mechanical behavior and/or movement of an element of the prosthesis 1, orthosis 2, or artificial limb 3, and a control means configured to control the force, resistance or motion generating device based on an adaptive model 8 (or control agent) and/or a detection model 7 (or state determining model). It is noted that the control means does not have to be a separate module and that it may be integrated with the processing means of the prosthesis 1, orthosis 2, or artificial limb 3. The configuration and/or behavioral data received from the secondary device 4 may be used to adapt the firmware and/or models used by the control means.

In an embodiment, the configuration and/or behavioral data are related to the operation of a force, resistance or motion generating device, such as an actuator or a damper, of the prosthesis 1, orthosis 2, or artificial limb 3. The actuator or the damper may affect the movement of a component of the prosthesis, orthosis, or artificial limb. During use, the actuator or the damper action may be defined based on a set of parameters affecting feedback loops for the control of the actuator or the damper. The configuration and/or behavioral data may comprise new values for at least a portion of the set of parameters in order to obtain a more natural motion of the prosthesis 1, orthosis 2, or artificial limb 3. Typically, the behavioral data will determine the external operation of the prosthesis 1, orthosis 2, or artificial limb 3, *i.e.* how the prosthesis 1, orthosis 2, or artificial limb 3 behaves with respect to the actions or activity the user is performing or in response to environmental properties (*e.g.* type of grounds, stairs, slopes, information about the person wearing the prosthesis or orthosis, or equipped with the artificial limb, etc.). The behavioral data may comprise software code, such as a control agent for controlling the operation of a force, resistance or motion generating device for influencing the mechanical behavior and/or movement of an element of the prosthesis 1, orthosis 2, or artificial limb 3.

The prosthesis 1, orthosis 2, or artificial limb 3 comprises at least one sensing means for obtaining sensed data related to the operation of the prosthesis 1, orthosis 2, or artificial limb 3 and/or to the environment of the prosthesis 1, orthosis 2, or artificial limb 3. The data storing means may store the sensed data and/or processed data based on the sensed data.

The control means may be configured to control the force, resistance or motion regulating device based on sensed data of the at least one sensing means in accordance with the control agent. Typically, the at least one sensing means may comprise multiple sensors, and the control means may be configured to select one or more sensors of said multiple sensors as an input of the control agent based on the configuration and/or behavioral data.

The at least one sensing means may comprise a first set of sensors and a second set of sensors; and the processing means may be configured to determine, depending on an activity mode of the prosthesis 1, orthosis 2, or artificial limb 3 in use, first processed data based on sensed data from the first set of sensors, and/or second processed data based on sensed data from the second set of sensors. In that way the data stored in the data storing means, and optionally transferred to the secondary device 4, may be adapted to be suited to the current activity mode. For example, depending on whether an individual is standing or walking or running, it may be desirable to sense different data and/or the process sensed data in a different manner.

Preferably, the communication module is connected through a wired connection to the prosthesis 1, orthosis 2, or artificial limb 3.

The control agent may receive one or more inputs and may use one or more weight parameters to generate one or more outputs based on the one or more inputs. The control agent may define a control policy, *e.g.* as known in the technical domain of artificial intelligence. For example, the one or more inputs may comprise outputs of one or more sensors of the at least one sensing means, and/or processed data based on the outputs of one or more sensors, and/or other data inputs. For example, the sensed data may further be processed in accordance with a detection algorithm to determine one or more operation categories of the prosthesis, *e.g.* an activity mode such as whether the person is walking on flat ground or on a slope, whether the person is standing, walking, or running, etc. These one or more operation categories may then be input in the control agent. The control agent may use one or more weight parameters associated with the different inputs. The one or more outputs of the control agent allow the control means to control the mechanical impact of the force, resistance or motion regulating device. The weight parameters may be used for adaptive control and different sets of weights can be defined based on the activity mode of the prosthesis 1, orthosis 2, or artificial limb 3 and based on the movement achieved by the prosthesis 1, orthosis 2, or artificial limb 3.

Optionally the control means may also output data to be stored in the data storing means in order to be transferred to the secondary device 4.

In an embodiment, the configuration and/or behavioral data may define one or more new sources of inputs for the control agent, and/or a new control agent itself, and/or one or more parameters used by the control agent.

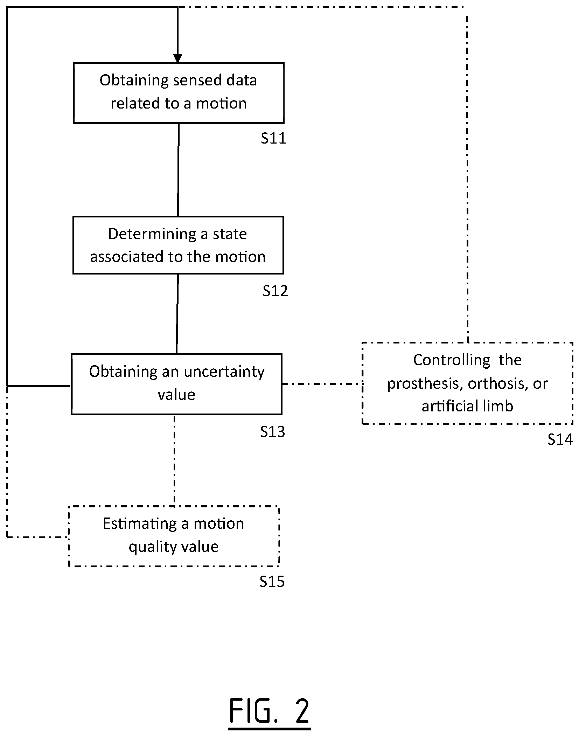

Figure 2 depicts a flow chart of an exemplary embodiment of a method for controlling a prosthesis, orthosis, or artificial limb, in a system comprising the prosthesis, orthosis, or artificial limb according to the present invention. The system comprising the prosthesis, orthosis, or artificial limb may be similar to the one described with respect to Figure 1.

In a first step S11, the method comprises obtaining sensed data related to a motion performed by a user involving the prosthesis, orthosis, or artificial limb worn or equipped by said user. The sensed data may be raw data which was directly obtained, *e.g.* from the at least one sensing means, pre-processed data based on the directly obtained data, or post-processed data. The at least one sensing means may be integrated or attached to the prosthesis, orthosis, or artificial limb and/or included in a wearable or mobile device.

The at least one sensing means may comprise any one or any combination of: a neural sensor, an angle-sensing means, an accelerometer, a gyroscope, a Hall sensor, a force sensor, a magnetometer, a pressure sensor, a torque sensor, a temperature sensor, an energy metering means, a current sensor, a voltage sensor, a humidity sensor, a sonar sensor, an EMG sensor, a barometric sensor, a grid of pressure sensor, an EEG sensor, a RFID sensor, a geo-localization sensor.

In addition to the sensed data obtained, other types of data may also be obtained. Another type of data related to the motion and involving the prosthesis, orthosis, or artificial limb may be data such as logs of control data used during use of the prosthesis, orthosis, or artificial limb, inputs to the processor of the prosthesis, orthosis, or artificial limb, physical characteristics of different elements composing the prosthesis, orthosis, or artificial limb, algorithms used for the operation of the prosthesis, orthosis, or artificial limb, software data for the processing means, user data (feedback) related to the felt performance, and structure data of the inputs to the model.

Depending on embodiments, sensed data may originate from sensing means that may be comprised by the prosthesis, orthosis, or artificial limb, said sensing means pertaining to the inner working of the prosthesis, orthosis, or artificial limb. Environmental data related to the environment of the prosthesis, orthosis, or artificial limb may be sensed data of sensing means comprised by the prosthesis, orthosis, or artificial limb, said sensing means pertaining to the surroundings of the prosthesis, orthosis, or artificial limb. The data related to the environment may relate to parts of the surroundings in which the user wearing the orthosis, prosthesis, or user equipped with the artificial limb is present or may relate to parts of the wearer or subject (*i.e.* user), such as data about a part of the body of the wearer or subject. In an embodiment, data related to the environment may be pertaining to a signal from a neural sensor implanted in the wearer of the prosthesis, orthosis, or the subject equipped with the artificial limb. Such signal(s) may include motion commands for the prosthesis, orthosis, or artificial limb.

The sensed data may be stored in a data storing means connected to the prosthesis, orthosis, or artificial limb. The first step S11 of obtaining sensed data may preferably be performed during use of the prosthesis, orthosis, or artificial. Complementary processed data may also be obtained at an ulterior time based on sensed data.

In a second step S12, the method comprises determining, by the state determining means, a state associated to the motion based on the sensed data. A trained model may be used by the state determining means to determine the state associated to the motion based on the sensed data. The state determining means may be integrated or attached to the prosthesis, orthosis, or artificial limb and/or included in a wearable or mobile device.

The state associated to the motion may correspond to a characterizable episode of the motion, the characterization of the episode considering any one or more of an intensity of the motion, a trajectory of the motion, a rhythm of the motion, or a duration of the motion. Therefore, depending on the accuracy, type, and/or the amount of sensed data, the state associated to the motion may correspond to the whole motion performed by the user involving the prosthesis, orthosis, or artificial limb, *e.g.* a throwing motion with a hand prosthesis, or may correspond to a more minute portion of the motion, *e.g.* the finger release of the object thrown with the hand prosthesis.

Depending on embodiments, the determining of the state associated to the motion may be achieved by determining a current event of the motion or by determining an activity related to the motion. More specifically, according to an exemplary embodiment, the determining of the state associated to the motion comprises determining an activity within a list of activities and/or a motion event within a list of motion events. Additionally or alternatively, instead of a list of discrete activities or a list of discrete motion events, the determining of the activity and/or the current event of the motion may be performed out of a continuum of activities and/or out of a continuum of motion events. Preferably, when determining the activity related to the motion, the state determining means uses one model per activity.

By activity, it is meant the type of motion implemented thanks to or via the prosthesis, orthosis, or artificial limb of the user. The activity may be defined based on a trajectory of motion, *e.g.* a gait pattern, a time duration of motion, and/or on various data sensed during the execution of the motion. The activity may also be associated with an activity level, *i.e.* how active the user is. The activity level may be determined *e.g.* based on the sensed motion. For example, in relation with the gait motion, the list of activities includes any one of the following: standing, jumping, walking (at different speed levels), running (at different speed levels), sitting, driving, using stairs, going at an incline (up or down), biking, laying down, etc.

Additionally or alternatively, motion activity analysis, *e.g.* gait activity analysis, may be performed based on the sensed data, and determining the activity related to the motion may be based on the motion activity analysis.

In an embodiment, forces analysis during motion, *e.g.* during the gait motion, may be performed based on the sensed data, and determining the current event of the motion may comprise determining a main phase of the motion and/or determining a subphase of the motion within the list of main phases and/or subphases of the motion.

According to an exemplary embodiment, the state determining means may be further configured to determine a plurality of state candidates, each of the plurality of state candidates being determined using a different state determining model. An uncertainty value, as will be explained more in details below with respect to step S13, may be obtained based on the plurality of state candidates. Also, instead of using only one model, the state determining means may use a plurality of models to determine the plurality of state candidates. Each of the plurality of models may be trained differently and may have different performances and precisions depending on a given state.

Following step S12, the method comprises a step S13 of obtaining, by the state determining means, an uncertainty value representative for a level of uncertainty that the user is in the determined state.

In the context of the present invention, the uncertainty shall be understood as a margin of error in a confidence interval which is typically calculated using the standard error of the point estimate, which is a measure of the variability of the sampling distribution of the estimate. The uncertainty is a concept that describes the level of confidence or doubt that one has in the outcome of a particular event or measurement. In an embodiment of the present invention, a model may be used by the state determining means to output the state associated to the motion of the prosthesis, orthosis, or artificial limb. In that case, the uncertainty value may be linked to the model output regarding the state. Because the model used may only have data from a sample of amputees, rather than the entire population, there will be some level of uncertainty associated with the estimate of a probability function of the states associated with the prosthesis, orthosis, or artificial limb.

The steps S11 to S13 described above are then iterated so that the state associated to the motion and the coupled uncertainty are continuously obtained through the use of the prosthesis, orthosis, or artificial limb.

Optionally, following step S13, step S14 may be performed. To do so, the system further comprises a control means. In step S14, the method further comprises the step of controlling, by the control means, the prosthesis, orthosis, or artificial limb based on the determined state and the obtained uncertainty value.

More specifically, the controlling of the worn or equipped prosthesis, orthosis, or artificial limb may comprise any one or more of the following: using, by the control means, a control agent to control an actuator of the prosthesis, orthosis, or artificial limb; controlling an adjustment of an element of the prosthesis, orthosis, or artificial limb.

The skilled person will understand that the steps S11 to S14 may be iterated during use by the user of the prosthesis, orthosis, or artificial limb so that a more accurate control of the prosthesis, orthosis, or artificial limb is achieved.

Optionally, following step S13, step S15 may be performed. In step S15, the method further comprises the step of estimating, by the state determining means, a motion quality value of the motion performed by the user, said motion quality value being based on optimal motion mechanical criteria. The motion quality value may correspond to how motion mechanical criteria of a certain motion performed by the user and involving the prosthesis, orthosis, or artificial limb scores compared to the optimal motion mechanical criteria for that certain motion.

More specifically, taking the example of the gait cycle for clarity-sake, sensed data related to the gait motion may be used for gait analysis in order to assess spatial, time, and temporal variables of the gait motion. These variables may include the limb movement and positions, joint angles, trajectories, velocities, generated force and muscle activity of particular body segments during the various phases of the gait cycle. Then, kinematic and biomechanical equations can be calculated to determine variations from known norms and establish a gait pattern of the user wearing the prosthesis or orthosis, or equipped with the artificial limb. It is known that each individual has a characteristic gait pattern. It can depend on a number of individual variables such as age, height, weight, sex, walking speed, strength, flexibility, type of surgery, shape of the amputation, and aerobic conditioning. The gait patterns can be assessed by conducting the gait analysis. The alterations in normal gait can be caused by different deformities, injuries, weakness, disease, or pain in any part of the body. A departure from the normal gait may be detected by estimating the motion quality value of the motion performed by the subject, a high motion quality value being associated to a normal gait.

The skilled person will understand that similar analyses may be performed for other motions or for prostheses, orthoses, or artificial limbs corresponding to other body parts. The skilled person will also understand that the steps S11, S12, S13, and S15 may be iterated during use by the user of the prosthesis, orthosis, or artificial limb.

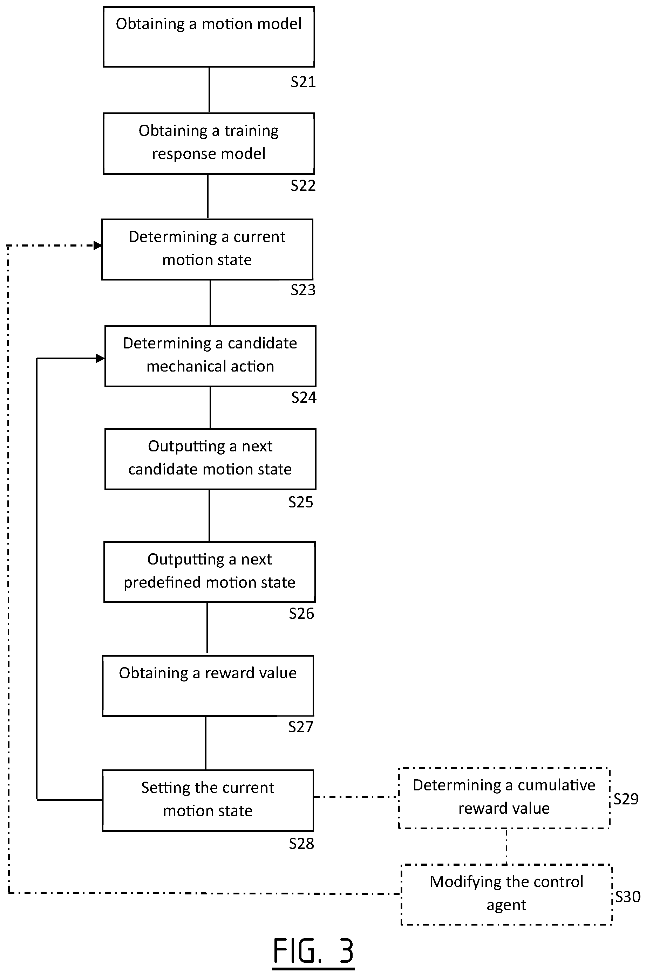

Figure 3 depicts a flow chart of an exemplary embodiment of a method for self-development of a control agent for a prosthesis, orthosis, or artificial limb according to the present invention. The prosthesis, orthosis, or artificial limb may be included in a system similar to the one described with respect to Figure 1.

In step S21, the method comprises the step of obtaining a motion model of the prosthesis, orthosis, or artificial limb, said motion model defining mechanical relationships between elements of the prosthesis, orthosis, or artificial limb.

By motion model, it is meant a virtual model designed to reproduce the prosthesis, orthosis, or artificial limb mechanically, from a sensor point of view, and also considering the controlling capabilities of said prosthesis, orthosis, or artificial limb. In an embodiment, the motion model may be implemented with a neural network.

In step S22, the method comprises the step of obtaining a training response model of the prosthesis, orthosis, or artificial limb, said training response model defining a predefined motion response of the prosthesis, orthosis, or artificial limb.

Preferably, the training response model is associated to the user wearing or equipped with the prosthesis, orthosis, or artificial limb. Additionally or alternatively, the training response model may be associated to an activity within a list of activities, and the list of activities includes any one of the following: walking, running, sitting, driving, using stairs, going at an incline, biking, laying down, standing, jumping.

Using the virtual reproduction of the prosthesis, orthosis, or artificial limb by the motion model, the predefined motion response would correspond to the desired mechanical behaviors of the prosthesis, orthosis, or artificial limb, said desired mechanical behaviors being associated to mechanical outputs of the prosthesis, orthosis, or artificial limb using sensed data as the inputs. The desired mechanical outputs are based on a plurality of natural equivalent responses of the user wearing or equipped with the prosthesis, orthosis, or artificial limb. The ensemble of natural equivalent responses from the subject wearing or equipped with the prosthesis, orthosis, or artificial limb are gathered in the training response model. The training response model may be acquired based on, for example, gait analysis of a healthy limb of the user, using an able-body subject, or using kinematic simulations based on physical characteristics of the user's body. The skilled person will understand that training response models may be obtained similarly for other body parts.

In step S23, the method comprises the step of determining a current motion state of the prosthesis, orthosis, or artificial limb. The current motion state may be determined depending on a desired starting point for the self-development of the control agent. Alternatively, the current motion state may be determined randomly within a list of motion states of a motion of interest.

In step S24, the method comprises the step of determining, by the control agent, a candidate mechanical action based on the current motion state, said mechanical action related to the elements of the prosthesis, orthosis, or artificial limb.

Complex relationships may be developed between sensed data as inputs and desired mechanical outputs. The ensemble of these complex relationships are gathered in the control agent, which may also be a model. Starting from an initial control agent, two aims are pursued for its development. One of the aims is to rate its performances by comparing the plurality of candidate mechanical actions through multiple iterations and their results with respect to the training response model using reward values. To achieve this aim, starting from the current motion state, the control agent will be left to proceed with determining the candidate mechanical action that should follow in order to reach the next logical motion state from the viewpoint of the control agent.

In an embodiment, the elements of the prosthesis, orthosis, or artificial limb includes an actuator, and the determining of the candidate mechanical action comprises determining a mechanical action of the actuator
In step S25, the method comprises the step of outputting, by the motion model, a next candidate motion state based on the current motion state and the candidate mechanical action.

The next logical motion state reached after applying the candidate mechanical action is called here the next candidate motion state. This next candidate motion state is obtained by taking into account the candidate mechanical action and how it affects the prosthesis, orthosis, or artificial limb thanks to the motion model, starting from the current motion state.

In step S26, the method comprises the step of outputting, by the training response model, a next predefined motion state based on the current motion state.

For a given motion and a starting current motion state, it is expected that there is a natural series of motion states. These motion states may be obtained from the training response model. The next predefined motion state may correspond to the following expected motion state that is desirable to be reached with a mechanical action from the prosthesis, orthosis, or artificial limb.

In step S27, the method comprises the step of obtaining a reward value based on the next candidate motion state and the next predefined motion state, said reward value being associated to the current motion state and the candidate mechanical action.

The next logical motion state, *i.e.* the next candidate motion state, may differ from the development of motion states as defined in the training response model. Comparing both, the reward value will be attributed to the couple constituted by the current motion state and the candidate mechanical action. In an embodiment, the reward value may be calculated by comparing the difference between the training response model and series of motion states obtained using the control agent.

In step S28, the method comprises the step of setting the next candidate motion state as the current motion state.

The steps S24 to S28 above will then be repeated from the new motion state reached, set up as the current motion state, thereby describing a series of motion states originating from mechanical actions and associated to reward values.

Optionally, following step S28, steps S29 and S30 may be performed. In step S29, the method further comprises the step of, upon reaching a predetermined condition, determining a cumulative reward function based on the obtained one or more reward values.

The iterations may continue until the predetermined condition is reached. In an embodiment, the predetermined condition may be a predetermined number of iterations. In another embodiment, the predetermined condition may be a convergence characteristic of the cumulative reward function obtained during the one or more iterations of the repeated steps. In yet another embodiment, the predetermined condition may be obtaining rewards values below a predetermined threshold in succession.

And in step S30, the method further comprises the step of modifying the control agent based on the cumulative reward function.

Another one of the aims of self-developing the control agent is to use the reward values which have been accumulated through the iterations so that the plurality of candidate motion states obtained from the plurality of candidate mechanical actions is closer to corresponding portions of the training response models. In this manner, a strategy is developed which will help in guiding the self-development of the control agent so that a series of mechanical actions ends up leading to a series of motion states mimicking a corresponding series of motion states from the training response model.

Depending on the cumulative reward function, the control agent may then be modified. If a score derived from the cumulative reward function is below a first score threshold, the first of the candidate mechanical action may be marked as a "dead-end" and the control agent may be made to start again going through multiple iterations from the same initial current motion state. If the score derived from the cumulative reward function is above the first score threshold or a second score threshold, then some parameters of the control agent may be reinforced so that similar mechanical actions as the candidate mechanical actions are employed when using the control agent with the control means of the prosthesis, orthosis, or artificial limb.

Preferably, the modifying of the control agent may comprise modifying a reward-grading algorithm used to obtain the one or more reward values during the one or more iterations of the repeated steps.

Depending on embodiments, the control agent may be developed to correspond to a single activity of the user, or can be developed to be adequate in being used for a continuum of motion states and activities.

The skilled person will understand that to continue the self-development of the control agent, after step S30, steps of the method can be repeated starting from step S23.

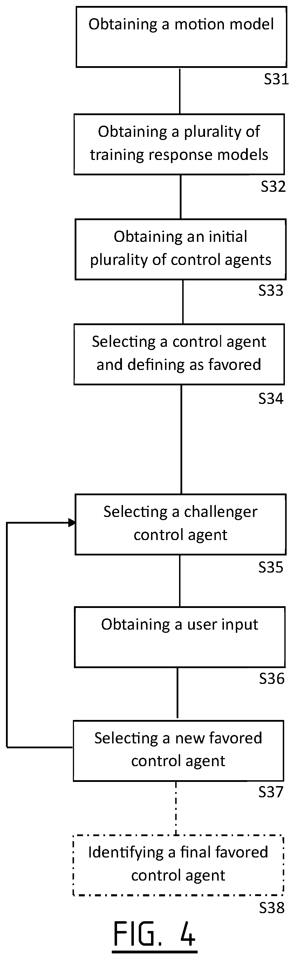

Figure 4 depicts a flow chart of an exemplary embodiment of a method for development of a favored control agent for a prosthesis, orthosis, or artificial limb according to the present invention. The prosthesis, orthosis, or artificial limb may be included in a system similar to the one described with respect to Figure 1.

In step S31, the method comprises the step of obtaining a motion model of the prosthesis, orthosis, or artificial limb, said motion model defining mechanical relationships between elements of the prosthesis, orthosis, or artificial limb.

In step S32, the method comprises the step of obtaining a plurality of training response models of the prosthesis, orthosis, or artificial limb. Each of the plurality of training response models defines a predefined motion response of the prosthesis, orthosis, or artificial limb and is associated to a biomechanically different user wearing or equipped with the prosthesis, orthosis, or artificial limb. Preferably, the plurality of training response models is associated to an activity within a list of activities, and the list of activities includes any one of the following: walking, running, sitting, driving, using stairs, going at an incline, biking, laying down, standing, jumping.

In step S33, the method comprises the step of obtaining an initial plurality of control agents, preferably similar control agents, each of the initial plurality of control agents being associated to a different training response model of the plurality of training response models, and configured for being used by a control means of the prosthesis, orthosis, or artificial limb in controlling the worn or equipped prosthesis, orthosis, or artificial limb.

The skilled person will understand that the initial plurality of control agents may be obtained by any known method. Alternatively or additionally, the initial plurality of control agents may be obtained by the self-development method described in the embodiment of Figure 3.

In step S34, the method comprises the step of selecting a control agent among the initial plurality of control agents, and defining the selected control agent as the favored control agent. The selection of the control agent among the initial plurality of control agents may be done randomly. Alternatively, the selection of the control agent may be done based on a comparison of physical characteristics of the user for which the control agent is developed with respect to physical characteristics of the plurality of biomechanically different users associated to the initial plurality of control agents.

In step S35, the method comprises the step of selecting a challenger control agent among the plurality of control agents, the challenger control agent being different from the favored control agent, and preferably not being selected in a previous iteration. The selection of the challenger control agent among the plurality of control agents may be done randomly.

In step S36, the method comprises the step of obtaining a user input indicative of a user preference among the challenger control agent and the favored control agent relative to a performance of the challenger and favored control agents, respectively, during use by the control means of the prosthesis, orthosis, or artificial limb in controlling the worn or equipped prosthesis, orthosis, or artificial limb. The obtaining of the user input may be achieved via a user interface of a mobile device.

In step S37, the method comprises the step of, based on the user input, selecting a new favored control agent between the previous favored control agent and the challenger control agent. The new favored control agent may be the higher rated by the user between the previous favored control agent and the challenger control agent.

The steps S34 to S37 above will then be repeated from the new favored control agent selected, set up as the favored control agent, thereby progressing the selection-by-competition within the plurality of control agents.

Optionally, following step S37, step S38 may be performed. In step S38, the method further comprises the step of, upon reaching a predetermined condition, identifying a final favored control agent. In an embodiment, the predetermined condition may include a predetermined number of selections of the same favored control agent during iterations of the repeated steps.

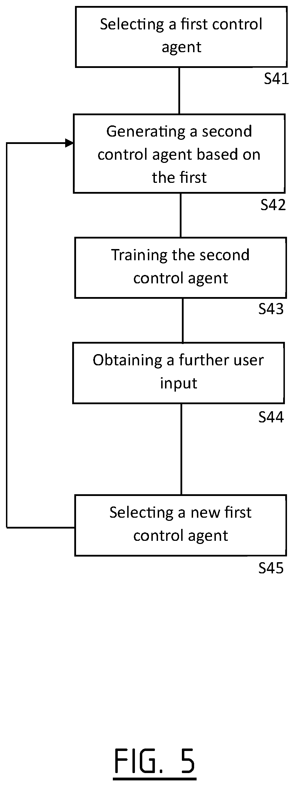

Figure 5 depicts a flow chart of an exemplary embodiment of a method for further development of a final favored control agent for a prosthesis, orthosis, or artificial limb according to the present invention.

In step S41, the method comprises the step of selecting the final favored control agent as a first control agent.

In step S42, the method comprises the step of generating a second control agent based on the first control agent, preferably by replicating the first control agent.

In step S43, the method comprises the step of training the second control agent based on the motion model of the prosthesis, orthosis, or artificial limb and the training response model associated with the first control agent, said training being preferably performed during a predetermined number of epochs.

In step S44, the method comprises the step of obtaining a further user input indicative of a further user preference among the first control agent and the second control agent relative to a performance of the first and the second control agents, respectively, during use by the control means of the prosthesis, orthosis, or artificial limb in controlling the worn or equipped prosthesis, orthosis, or artificial limb. The obtaining of the further user input may be achieved via a user interface of a mobile device.

In step S45, the method comprises the step of, based on the further user input, selecting a new first control agent between the previous first control agent and the second control agent. The new first control agent may be the higher rated by the user between the previous first control agent and the second control agent.

The steps S42 to S45 above will then be repeated from the new first control agent selected, set up as the first control agent, thereby progressing the development-by-training with respect to a prior replicate of the first control agent. The skilled person will understand that the new first control agent may be used at any iteration by a control means of the prosthesis, orthosis, or artificial limb in controlling the worn or equipped prosthesis, orthosis, or artificial limb.

Whilst the principles of the invention have been set out above in connection with specific embodiments, it is to be understood that this description is merely made by way of example and not as a limitation of the scope of protection which is determined by the appended claims.

## Claims

1. A method for development of a favored control agent for a prosthesis, orthosis, or artificial limb, comprising the steps of:
- obtaining a motion model of the prosthesis, orthosis, or artificial limb, said motion model defining mechanical relationships between elements of the prosthesis, orthosis, or artificial limb;
- obtaining a plurality of training response models of the prosthesis, orthosis, or artificial limb, each of said plurality of training response models defining a predefined motion response of the prosthesis, orthosis, or artificial limb and being associated to a biomechanically different user wearing or equipped with the prosthesis, orthosis, or artificial limb;
- obtaining an initial plurality of control agents, preferably similar control agents, each of the initial plurality of control agents being associated to a different training response model of the plurality of training response models, and configured for being used by a control means of the prosthesis, orthosis, or artificial limb in controlling the worn or equipped prosthesis, orthosis, or artificial limb;
- selecting a control agent among the initial plurality of control agents, and defining the selected control agent as the favored control agent;
wherein the method further comprises the steps of, repeatedly:
- selecting a challenger control agent among the plurality of control agents, the challenger control agent being different from the favored control agent, and preferably not being selected in a previous iteration;
- obtaining a user input indicative of a user preference among the challenger control agent and the favored control agent relative to a performance of the challenger and favored control agents, respectively, during use by the control means of the prosthesis, orthosis, or artificial limb in controlling the worn or equipped prosthesis, orthosis, or artificial limb;
- based on the user input, selecting a new favored control agent between the previous favored control agent and the challenger control agent.

2. The method according to claim 1, wherein the method further comprises the step of:
- upon reaching a predetermined condition, identifying a final favored control agent.

3. The method according to claim 2, wherein the method further comprises the steps of:
- selecting the final favored control agent as a first control agent; and
wherein the method further comprises the steps of, repeatedly:
- generating a second control agent based on the first control agent, preferably by replicating the first control agent;
- training the second control agent based on the motion model of the prosthesis, orthosis, or artificial limb and the training response model associated with the first control agent, said training being preferably performed during a predetermined number of epochs;
- obtaining a further user input indicative of a further user preference among the first control agent and the second control agent relative to a performance of the first and the second control agents, respectively, during use by the control means of the prosthesis, orthosis, or artificial limb in controlling the worn or equipped prosthesis, orthosis, or artificial limb;
- based on the further user input, selecting a new first control agent between the previous first control agent and the second control agent.

4. The method of any one of claims 1-3, wherein the obtaining of the initial plurality of control agents is based on one or more sensed data from a list of available sensed data during use of the prosthesis, orthosis, or artificial limb.

5. The method of claim 4, wherein the list of available sensed data comprises data sensed by any one or more of the following sensing means: a neural sensor, an angle-sensing means, an accelerometer, a gyroscope, a Hall sensor, a force sensor, a magnetometer, a pressure sensor, a torque sensor, a temperature sensor, an energy-metering means, a current sensor, a voltage sensor, a humidity sensor, a sonar sensor, an EMG sensor, a barometric sensor, a grid of pressure sensors, an EEG sensor, a RFID sensor, a geo-localization sensor.

6. The method of any one of claims 2-5, wherein the predetermined condition includes a predetermined number of selections of the same favored control agent during iterations of the repeated steps.

7. The method of any one of claims 1-6, wherein the plurality of training response models is associated to an activity within a list of activities, and wherein the list of activities includes any one of the following: walking, running, sitting, driving, using stairs, going at an incline, biking, laying down, standing, jumping.

8. A system comprising a prosthesis, orthosis, or artificial limb, said system further comprising:
- at least one sensing means configured to sense data related to a motion performed by a user involving the prosthesis, orthosis, or artificial limb worn or equipped by said user;
- a control means configured to use the favored control agent developed according to the method of claim 1 in controlling elements of the prosthesis, orthosis, or artificial limb.

9. The system of claim 8, further comprising at least one user interface device configured to receive a user input.

10. The system of claim 8 or 9, wherein the control means is integrated or attached to the prosthesis, orthosis, or artificial limb, and/or included in a wearable device or mobile device.

11. The system of any one of claims 8-10, wherein the at least one sensing means is integrated or attached to the prosthesis, orthosis, or artificial limb, and/or included in the wearable device or the mobile device.

12. A computer program comprising instructions which, when the program is executed by a computer, causes the computer to carry out the steps of the method according to any one of claims 1-7.
